# EUROPEAN PATENT APPLICATION

(11) **EP 4 814 874 A1**
(43) Date of publication of application: **30.09.2026**
(21) Application number: 25305434.0
(22) Date of filing: 25.03.2025
(51) Int. Cl.: G06T 11/00, A61B 6/00

(54) **AUTOMATIC 3D X-RAY VIEW GENERATED DURING USAGE OF THE X-RAY SYSTEM**

(71) Applicant: Koninklijke Philips N.V., 5656 AE Eindhoven (NL)
(72) Inventor: HENDRIKS, Bernardus, 5656 Eindhoven (NL); SCHAEFER, Dirk, 5656 Eindhoven (NL); ABDOLI, Mehrsima, 5656 Eindhoven (NL); VAN DER HORST, Tim, 5656 Eindhoven (NL); LELY, Hein, 5656 Eindhoven (NL); PETERS, Inge, 5656 Eindhoven (NL); MOESKOPS, Jonne, 5656 Eindhoven (NL); ELENBAAS, Thijs, 5656 Eindhoven (NL); FLORENT, Raoul, 5656 Eindhoven (NL)
(74) Representative: Philips Intellectual Property & Standards

(57) **Abstract**

System (SYS) and related method for imaging. The system comprises a projection image accumulator (ACC) causing projection imagery (*λ,λₜ*) acquired by an interventional X-ray based imaging apparatus (IA) to be accumulated in a memory (MEM) whilst at least some such projection imagery (*λ*) is being displayed on a display device (DD) for image-based guidance of a user in performing a procedure in respect of a region of interest. A reconstructor (RECON) is capable of reconstructing at least one reconstructed image (*m,mₜ*), based at least on some of the accumulated projection imagery.

## Description

### FIELD OF THE INVENTION

The invention relates to a system for imaging, to a related method, to an imaging arrangement including such as system, to a computer program element, and to a computer readable medium.

### BACKGROUND OF THE INVENTION

Cardiac afflictions of all manners are still the leading cause for premature death, degradation of quality of life, etc. As per an 11 June 2021 factsheet (available online at https://www.who.int/news-room/fact-sheets/detail/cardiovascular-diseases-(cvds)) by the WHO (World Health Organization), the toll due to CVD (cardiovascular disease) stands at 17.9 million premature deaths worldwide.

Modern medicine provides a range of options, some of which are imaging based. For example, fluoroscopy or angiography may be used that provide live 2D imaging. It allows visualization of anatomic aspects, but also of devices or tools needed in an intervention, such as catheter, stents, etc. Percutaneous coronary interventions (PCI) in a "cath lab" (catheterization laboratory) are one example, where a cardiac stenosis may be treated.

Some projection imaging based imaging apparatuses ("imager"), such as of the C-arm type, allow obtaining 3D X-ray views in the "cath lab" (catheterization laboratory) that may enable better guidance, eg when advancing interventional devices through complex structures in the body. Generating 3D X-ray views in the cath lab is however time consuming, and disrupts user workflow with associated distractions: for example, such 3D imaging requires imager's gantry (eg, C-arm) be repositioned, well iso-centrically, and distractive attention needs to be paid during such rotational acquisition for this 3D imaging purposes, for example to ensure that there are no obstructions in the rotating gantry's path, etc.

### SUMMARY OF THE INVENTION

There may therefore be a need for improved X-ray based imaging.

An object of the present invention is achieved by the subject matter of the independent claims where further embodiments are incorporated in the dependent claims. It should be noted that the following described aspect of the invention equally applies to the related method, to the imaging arrangement, to the computer program element, and to the computer readable medium.

According to a first aspect of the invention there is provided a system for imaging, comprising:
a projection image accumulator causing projection imagery acquired by an interventional X-ray based imaging apparatus to be accumulated in a memory whilst at least some such projection imagery is being displayed on a display device for image-based guidance of a user in performing a procedure in respect of a region of interest; and
a reconstructor capable of reconstructing at least one tomographic image, based at least on some of the accumulated projection imagery.

In embodiments, the system operates in the background during an imaging session with image-based guidance.

In embodiments, the said reconstructing is ongoing whilst some of the projection imagery is so being displayed.

In embodiments, the reconstructed image is displayed once one or more image quality, IQ, condition is met as establishable by an IQ checker.

In embodiments, the said IQ condition is based on back projections of the stored projection imagery and/or is based on segmentation of the projection imagery.

In embodiments, the said one or more IQ condition is based on segmentation of the projection imagery.

In embodiments, the reconstructed image is of a region other than the ROI.

In embodiments, any one of initiation of i) reconstruction and ii) the displaying of the reconstructed image is done automatically.

In embodiments, different plural such images are reconstructed at different field of views, FOVs based on respective different set of projection imagery acquirable over time.

In embodiments, the imaging geometry is adjusted for acquisition of the projection imagery.

In embodiments, imaging geometries are not confined to a plane in 3D.

In embodiments, further projection imagery is accumulated, and the reconstructed tomographic image is refined by accounting for said further projection imagery in a follow up reconstruction operation by reconstructor.

In embodiments, the reconstructor is configured to apply a tomographic reconstruction algorithm for sparse reconstruction.

In embodiments, the imaging apparatus is a C-arm imager and/or is configured for fluoroscopy.

In embodiments, the procedure includes performing a remedial action in respect of the ROI and/or one or more prior preparatory actions in preparation of such remedial action.

In embodiments, the ROI includes at least part of a vasculature of a subject.

In embodiments, the projection imagery are stored in said memory in association with respective data indicative of the relevant imaging geometry for the respective projection image.

In another aspect there is provided a medical imaging arrangement including system and at least one of: the display device, the imaging apparatus, the said memory.

In another aspect there is provided a method for imaging, comprising:-
storing projection imagery acquired by an interventional X-ray based imaging apparatus in a memory, whilst at least some such projection imagery is being displayed on a display device for image-based guidance of a user in performing a procedure in respect of a region of interest; and
reconstructing at least one tomographic image *(m),* based at least on some of the accumulated projection imagery.

In another aspect there is provided a computer program element, which, when being executed by at least one processing unit, is adapted to cause the processing unit to perform the method.

In another aspect there is provided at least one computer readable medium having stored thereon the program element.

In the proposed setup, the projection imagery so stored and displayed may not necessarily and always pertain to the ROI itself only. The projection imagery may pertain to other regions, at least at times. This is because the procedure may pertain, that is, its purpose is, indeed in relation to the ROI, but the procedure may proceed over plural phases, in which other regions may be imaged. For example, in PCI, a tool (catheter, guide wire) may need to be navigated first to the ROI, thereby passing other regions, and these are imaged in the projection imagery first.

A problem address herein is how to obtain 3D X-ray images in interventional imaging, which require no additional, or minimal, or at least reduced, handling or attention by user.

The proposed 3D imaging runs along 2D imaging in an automated manner, without user attention. It may be thought of proceeding in a "random manner", alongside the 2D projection imaging. As such, in envisaged scenarios, user does "not care" about the 3D reconstruction. User mainly focuses on the 2D projection images that are to provide live action feed/image stream for image guidance. The 3D reconstructions are produced "along the way". They are reconstructed from whatever useful projection frames the accumulator system can pick up from the live stream. Once a good enough 3D reconstruction is computed, this is made to "pop up" on screen. Thus, what is proposed herein is a "random" harvesting for reconstruction of the image-guidance conferring acquired projection images. Thus, there is no dedicated acquisition of projection images solely for reconstruction. The system is primarily for projection imaging (with variable imaging geometry) and provides now and then, when certain image quality ("IQ") standard(s) is met, some reconstructed imagery for additional 3D insight. The imaging geometry may be adjusted by user to "follow" the procedure, which may be dynamic such as moving a tool/device towards the ROI. The user thus causes the projection imagery stream to be acquired for the purpose of 2D image guidance. No thought need be spared by user to the 3D imaging. This is done in the background. 3D imagery is reconstructed now and then from the collected projection imagery that happen to clear certain IQ quality checks. Otherwise, the system remains mute and keep operating in the background attempting so selected from the stored projection imagery certain frames that together allow reconstruction at the requisite image quality.

In embodiments, during an interventional procedure performed with an X-ray system, various 2D X-ray projection images are taken at different imaging geometries, such as at different positions and/or angles of a C-arm. In existing images, these live action frames so far have not been used for further purposes. In departure from this, we propose herein to accumulate/store these 2D projection images, preferably together with their C-arm position (location and angulation) or other imaging geometry information. From these stored 2D projection images, and from corresponding imaging geometry data, such as coordinates etc, a 3D image reconstruction may be done. For such reconstruction, a sparse tomographic reconstruction is preferably used, as in general there are no projection images over a full 180° arc available. Different reconstructions are repeatedly (quasi-constantly) performed as a computing background process, and the results are presented to the user on display device during use of interventional imager.

The reconstruction may only be done once certain quality checks are passed to save CPU time. For example, for a decent 3D there should be at least two, better at least three, different views along different directions available, preferably spaced sufficiently apart. The stored imaging geometry data may be used for establishing this, and/or back projection from the available 2D projection data.

*"user"* relates to a person, such as medical personnel or other, operating the imaging apparatus or overseeing the imaging procedure. In other words, the user is in general not the patient.

*"subject"* is used herein in the general sense to include animate *"subject"* such as a human or animal patient, or anatomic parts thereof but also includes inanimate subjects such as an item of baggage in security checks or a product in non-destructive testing. However, the proposed system will be discussed herein with main reference to the medical field, so we will be referring to the "subject" as "the patient" or a part of the patient, such as an anatomy or organ, or group of anatomies or organs of the patient.

As to notation used herein, designation "*m*" will be used herein consistently to refer solely to tomographic imagery/image, that is, to the result of a tomographic reconstruction operation from plural multi-directional projection images "*λ*". Thus, a short-hand reference to *"image(s)*/*imagery m"* is appropriate and will used herein at times." *λ*" on the other hand will be used herein consistently as notation for projection imagery. Projection imagery refers in general to plural frames. In order to refer specifically to the time series character or to a given image or frame, notation *λt: = λ(t), mt: =m(t)* may be used at times. The reconstructed imagery may include a slice or a volume of voxels.

### BRIEF DESCRIPTION OF THE DRAWINGS

Exemplary embodiments of the invention will now be described with reference to the following drawings, which, unless stated otherwise, are not to scale, wherein: -
Fig. 1 is a schematic drawing of an imaging arrangement;
Fig. 2 are illustrations of imaging geometry as pertains to imaging operations of an imaging apparatus;
Fig. 3 shows a block diagram of a facilitator system that may facilitate 3D and 2D imaging;
Fig. 4 illustrates forward- and backward projection operations as may pertain imaging as envisaged herein in some embodiments; and
Fig. 5 shows a flow chart of a computer implemented method of facilitating, in particular X-ray based 2D and 3D imaging.

### DETAILED DESCRIPTION OF EMBODIMENTS

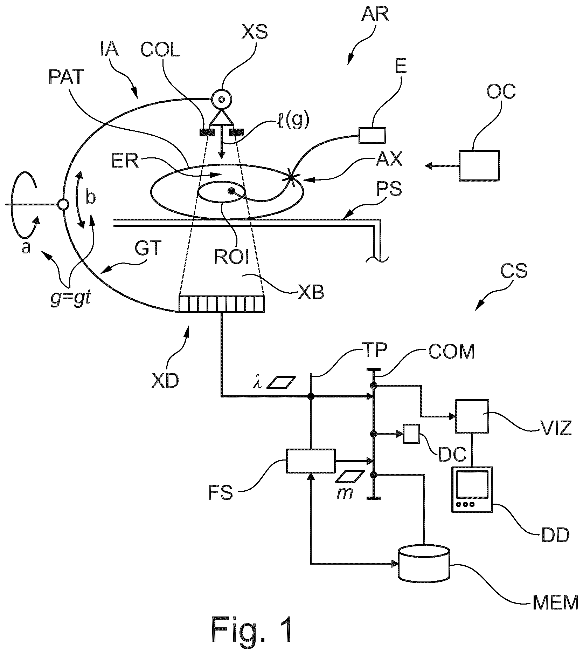

Reference is made first to the block diagram of Fig. 1, which schematically shows components of an imaging arrangement AR. In particular, the imaging arrangement AR may be set up as a medical imaging arrangement, and this is mainly envisaged herein, although applications of the imaging arrangement are not necessarily confined to the medical field. The imaging arrangement AR may include an imaging apparatus IA and a computing system CS, the latter capable of processing data it receives from the earlier.

Briefly, and as will be expanded upon more fully below, the imager is capable of multiple imaging modes, in particular of 2D and 3D imaging, and the computing system CS includes functionality FS to handle or coordinate, in an automated manner, the said different imaging modes.

Specifically, the imaging arrangement AR may include an interventional X-ray imaging apparatus IA capable of i) allowing an interventionalist (human or robot) access to an imaged subject PAT, and ii) acquiring spatial projection data λ along multiple spatial directions relative to a reference coordinate system. Such imaging apparatus ("imager") may include an imager IA of the C-arm or U-arm type, or similar. Such C-arm type imager IA, as schematically indicated in the Figure, has the eponymous C-shaped gantry GT. Gantry GT forms a cutout, thus affording to medical user such access, and the C-arm GT, which carries imaging components such as X-ray source XS and X-ray detector XD, is movably mounted in order to realize said multi-directional imaging capability. Different spatial field of views (FOVs) can be so realized. Thus, the imager IA envisaged herein is not a (purely diagnostic) CT scanner with doughnut shaped gantry.

The computing system CS may be data-commutatively coupled, via wireless, wired or hybrid communication infrastructure COM, to imager's detector XD in order to receive therefrom, and process, the spatial data λ acquired by the imaging apparatus. The computing system CS may be partially or wholly integrated into the imaging apparatus, such as in imager IA's operator console OC, or in other computing system, node or device, or at least the computing system may be arranged partly or wholly in the same exam room in which the imager IA is present. In some embodiments, some of the proposed functionalities of the computing system may be arranged remotely, such as 3D reconstruction functionality. Said spatial data as produced by the imaging apparatus IA may be in the form of 2D projection imagery λ. The projection imagery can be visualized by a visualizer VIZ on a display device DD during interventional use of imager IA, for example during user (human or robot) performing a procedure in respect of a region of interest ROI of imaged subject PAT, such of a patient. For example, the region of interest (for the procedure and/or the imaging) may be a lesioned site, such as stenosis in a vasculature of the subject's heart. The procedure may be one of angioplasty to relieve stenosis, but numerous other procedures are envisaged herein.

One or more tool or device E may be introduced through an access point AX into subject PAT, in order to perform or support the procedure. The procedure may proceed over multiple phases, at least one such phase effecting a remedial action in respect of the ROI, such as alleviating the lesion, eg, broadening the stenosis by placing a stent and/or by operation of balloon catheter, or others.

The projection imagery is provided as a stream of such projection images, referred to as frames herein, that, when displayed/visualized in time sequence, provide a live action video feed. Such feed can be visualized on a display device DD by streaming the projection imagery from detector XD through the communication structure COM to the display device for displaying said video feed, or of still images/frames, as needed. Thus, the imaging arrangement allows user to observe via said live imagery (video or still) internal structures within subject PAT, such as the ROI, intermediate or other anatomical structures R, and the (one or more) tool E itself. In this manner, the imaging arrangement is capable of providing 2D image-guided support to user/procedure.

As mentioned, the procedure may proceed over many phases, such as introduction phase, navigation phase where the device E is navigated towards the ROI through an intricate system of conduits, such as the vasculature. The tool, a catheter, guidewire, etc, may be navigated towards the stenosis in the patient's cardiac vasculature. For example, the user can observe anatomies that are passed allowing the way as the elongated tool E is threaded towards the region of interest, whilst the live action feed of projection imagery is acquired and displayed in real-time on display device DD, thus providing (live-) image-guided support. For example, the user can see on the display real time video that visualizes how the device E is moving, its state is changing, such as its expansion, and also certain physiological activities, such as heart activity or respiratory activities, or other (subsidiary) one or more activity caused by such prime activity.

The set-up of the X-ray imaging apparatus being of the C-arm type or similar, is such that it enjoys a possibly large number of degrees of freedom of adjusting an imaging geometry of the imager so that projection imagery can always be acquired at a most opportune spatial direction ℓ. The spatial direction is a function of the imaging geometry used. The field of view (FOV) is a function of imaging geometry. The imaging geometry, and thus the FOV, may be adjusted potentially many times over during the procedure, depending on imaging context, such as the current region (which may not be the ultimate /actual ROI for the clinical purpose of the procedure), a given portion of an anatomy, phase of procedure, state (expanded/or not) or position, etc, of device E, etc.

Whilst the imaging apparatus is primarily envisaged herein for 2D imaging, such as the said projection imaging, it may also allow, in addition, in a different mode of operation, tomographic imaging . In tomographic imaging, (3D) tomographic imagery *m* (either a single slice, multiple slices, or a volume(s)) may be obtained, for yet better image-guided support of user, or other use, as needed. 2D Projection imaging is still one of the most preferred modes of imaging, due to its simplicity, but of course, it suffers from a certain loss of spatial information in respect of structures that happen to overlap, one or more behind one or more others, along a given projection direction. Their attenuative contributions as recorded by the relevant image pixels at the detector XD are "collapsed", summed as line integrals, into the same image values in projection domain. Tomographic imagery on the other hand allows a 3D view "behind" such structure(s), thereby avoiding or reducing such spatial information loss. Spatial ambiguities are resolved the larger the angular range, ideally 180°, for which projection imagery is available. But even an angular range less than 180° can reveal sufficient spatial insight for a given task at hand, at any rate more such spatial insight than a projection image along a single direction can. Thus, 3D imaging can usefully supplement 2D imaging, for better understanding of functional or structural constitution of the imaged locales, thus can better inform the procedural needs in particular for therapy, but also around aspects of diagnosis and/or planning.

A main purpose for 2D projection imaging as envisaged herein is dynamic real-time image-guided support. The requirements for such 2D projection imaging may change from procedure to procedure, or may vary from phase to phase of the same procedure. In distinction to 2D imaging, the 3D imaging is done herein not primarily in real-time, but supplemental, and in an automated way, alongside the more user activity consuming 2D imaging. The 2D projection imagery and 3D imagery is primarily used for visual consumption by user. Other data consumption of either the 2D imagery or the 3D imagery are not excluded herein, such as processing such imagery for diagnostic or planning purposes or other, rather than for image-guided support as primarily envisaged herein. For example, a data consumer DC, such as an artificial intelligence diagnostics system, may run in parallel to examine the live action feed projection imagery λ and/or the occasional 3D imagery.

What is proposed herein is the computing system to include a facilitator FS system, a computational entity or node or software program, that is operable to handle both modes, the X-ray imaging mode, in particular for acquiring projection imagery, and the 3D tomographic imaging mode. Briefly, and as will be elaborated below in more detail, the facilitator system FS is operable to compute occasional tomographic imagery *m* from the received and viewed/displayed projection images *λ* (=*λt*)*.* Such occasional tomographic imagery *m* is produced by a tomographic reconstruction only when certain one or more conditions, such as in terms of image quality and/or others, are met. The operation of tomographic reconstruction may be considered a computational expenditure in terms of memory and processor (CPU, GPU, etc.) time, which is preferably only expended (in full) once the one or more IQ criteria are met. It may happen that the image quality is only ascertainable once at least part of the reconstruction concluded. In this case, if such post-reconstruction in-image-domain quality check fails, the reconstructed image *m* is not displayed at all, so as not to cause undue distraction of user. In this case, such low quality imagery *m* may be discarded, expunged from memory, or may still be retained in memory, whilst not (yet) automatically displayed. Such retained low quality imagery *m* may be used for other purposes, such as radiology staff training, audits, etc, or may be used as initial data for a new run of reconstruction once more projection data become available. Thus, the proposed quality check may be two-stage, prior reconstruction in projection domain and post-reconstruction in image domain. Alternatively, a simple 1-stage embodiment with operation in either domain, but not both, is also envisaged, and so is a user-configurable setup where user specifies the type of IQ check to run, either 2- or 1-stage, and, if the latter, in which domain.

The facilitator FS may be operable purely in software or purely in hardware, or partly both. The facilitator FS may operate in the background, such as a background process, for example, run on a suitable computing system such as on the supporting computing CS. Such computing system CS may be included or may be part of, or may indeed be identical to, the computing system that implements the operator console OC, for example. Operation of the facilitator FS run on whichever computing system may largely pass as un-noticed by the user, unless when the said certain image quality conditions are met, at which point or shortly thereafter, the reconstructed imagery, reconstructed in the background, is brought up and its displaying is effected, either on the same display device on which the projection imagery is viewed, or on a different display device. Whilst the reconstructed imagery is based on some of the acquired and displayed projection imagery, displayed so as to provide the image-guided support to user during to procedure, some such projection imagery may be unrelated to the reconstruct imagery.

The 2D projection imagery stream *λ* =*λt*, and the occasionally, at certain times t' reconstructed image(s) *mt',* may be displayed concurrently. The user may switch between the two views, when indeed a reconstructed tomographic image *m* is available for display at a given time *t'.* Such availability can be indicated to user by an alert functionality in whichever form, such as visual, haptic, or audio. Indeed, such alert in other than visual form may be preferred so as not overload the screen. This is because the function of display device is primarily for user to follow the projection imagery based live action video feed (or still imagery). Thus, user can focus on monitoring with due attention and confirm whether procedure is unfolding to plan. An audio signal may be sounded out, or haptic activation of imager's user control interface of operator console OC may be effected. Such user control interface (UI) may be of the electro-mechanical ("e/m")-type, such as in the form of a joystick or pedal or similar, through which user initiates imaging and/or requests imaging geometry adjustments. Handles or footrest of such e/m-UI may be set into vibration to so notify user on availability of imagery *m* for view. Alternatively, or in addition, flashing light is activated away from the screen. Having said that, in some embodiments the screen may still present an animated, eg flashing or other, GUI widget (element) to indicate to user said availability. Even less obtrusive notification on the screen may also be done instead of or in addition to the above, such as via inclusion in screen of textual matter, flag, or other.

On such notification, in whichever form, of image *m* availability, the user may input via suitable input interface, such as physical or on-screen GUI widget, an "of-interest" signal. Once such signal is issued and registered, the visualizer VZ is instructed by facilitator FS to cause the actual displaying of the available image *m.* In other, more fully automated embodiments, the tomographic image *m,* once reconstructed and available, is caused to be displayed automatically on screen of the display device, such as in a gradual fade-in, or at once in a pop-up, or at a designated portion of display device's screen, etc. in such automated embodiment, no user confirmation for effecting displaying is needed.

It should be noted that during the procedure there may be plural such different images *mt'* brought up at different times t' once reconstructed and quality-checked, that may pertain to different portions of image domain/of subject, with a different field of view. Some or each may pertain to different regions *R=Rt'* ← *mt',* which may well differ from the ROI for the purpose of the procedure. For example, some such reconstructed images *mt'* may be brought up for displaying during the navigation phase, whilst the user navigates the tool E towards the lesioned site ROI. On its way there, during the image-based guidance of the user, the live action feed of projection imagery λ is displayed, regardless of which image is reconstructed and available. This is because the projection imagery λ is acquired for the purpose of providing live image guided support for user throughout the procedure. The reconstructing of the tomographic imagery *m* is subsidiary to this. If it happens to be the case, more or less by chance as it where, that the one or more quality condition is met at some point in time *t'* during, say, the navigation phase, the reconstruction is done and the respective image *mt'* may then be displayed, either automatically or when user sanctioned. Thus, the functionality of facilitator FS may have various automated aspects: any one or more, indeed preferably all of the following may be effected automatically without user input: i) accumulation in non-volatile memory of acquired and display projection imagery λt, ii) the quality check in either or both stages, iii) the reconstruction itself, and iv) the displaying operation. In some less automated embodiments, some one or more of such aspects, in particular aspect iv), may be done based on some user input, as explained above. As to the IQ condition(s), these may be formulated based various polices, which may include one or more of a) sufficient number or projection imagery at sufficient quality, or b) back-projections of some of the stored happen to have sufficiently defined spatial intersection in image domain, or c) other such or similar conditions still.

Before explaining in more detail operation of the imaging apparatus, in particular of the facilitator system FS, further imaging context will be provided first on various application scenarios as envisaged herein, and the operation of the imaging apparatus and its components in order to facilitate later in-depth explanations.

The interventional imaging apparatus IA includes the X-ray source XS and the X-ray sensitive detector XD. The X-ray source XS, and opposite thereto, the X-ray sensitive detector XD, are arranged preferably on the rotatable gantry GT. The gantry GT may have a recess in which an examination region ER is defined where the patient PAT, or in particular the region of interest, resides during imaging.

Generally, imaging includes energizing the X-ray source XS, so that an X-ray beam issues forth from a focal spot of the source XS, traverses the examination region ER (with the subject PAT in it) and interacts with patient tissue matter. Interaction of X-ray beam XB with tissue matter causes the beam to be modified. The modified beam may then be detected at the detector XD. DAQ/conversion circuitry (not shown) converts detected intensities into digital imagery, in particular into the projection imagery λ. The patient PAT may reside on a patient support PS such as a table during imaging.

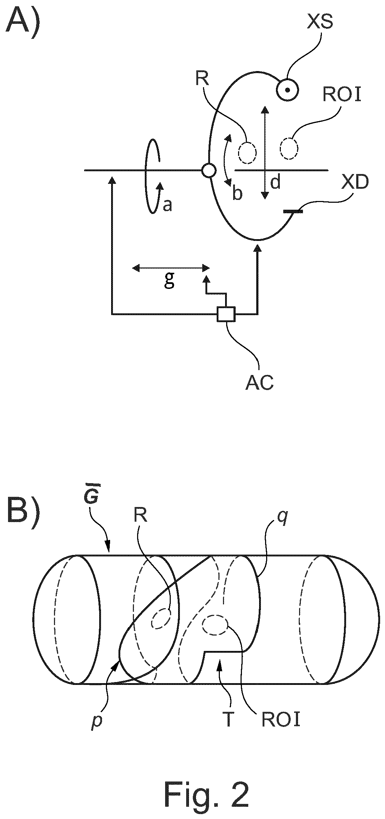

Reference is now made to the schematic drawings in Figs 2A), B) which explain in more detail the adjusting of imaging geometry g as envisaged herein in embodiments, in order to realize acquisition of different projection imagery at different FOV views. The imaging geometry g, which may change over time *g*=*gt*=*g(t)* , may be function of user request via control interface (operator console joystick, or other). The imaging geometry may vary as a function of procedure type, type of interventional device E used, phase of procedure, anatomical variation or peculiarities encountered during the interventional procedure, etc.

Fig. 2A) schematically represents various degrees of freedom (DoFs) of adjusting the imaging geometry in a C-arm imager IA. In more detail, spatial aspects of imaging geometry (see also with continued reference to Fig. 1) may be represented as pose (orientation and/or position) in 3D space of imaging line *ℓ* = *(g)* in the imaging domain/examination region ER. The imaging domain ID/region ER is the portion of space between the X-ray source XS and the X-ray detector XD, both held by the C-shaped gantry. The imaginary imaging line *ℓ* may run from a center point of imaging surface of the detector XD to a focal spot of source XS. The imaging line *ℓ* may be related to the general projection direction/view at a given imaging geometry setting. Other spatial aspects may further include shape/width of beam, as may manipulated by setting radiation-opaque blades of optional collimator COL. So setting the collimator blades may form an aperture for the beam to pass through. Other spatial aspects may include the length of line *ℓ* , which corresponds to SID parameter δ.

The various DoF as pertains to imaging geometry *g* = *g*(α,...,δ).*)* may be parameterized by parameters (α,...,δ). Such imaging geometry parameters (α,...,δ) may pertain to angulation α , rotation β , shift/translation γ, and said source detector (SID) distance δ. Some of the parameters (α,...,δ) may parameterize line *ℓ* in space 3D, and hence at least partly define respective instantaneous FOVt= FOV. Any one or more, in particular all, of the parameters (α,... ,δ) may be adjusted independently by operation of a set of actuators or effectors AC, such as server motors, stepper motors, or others, to so effect changes in the imaging geometry and to then acquire, once imaging geometry is reset, respective projection frames in the desired field of view. The imaging geometry, and hence pose of line *ℓ,* its length etc, may well be changed many times over during the procedure, in particular, as the procedure passes through its different phases. The actuators AC may act on C-arm GT as it is held in suitable bearings, journalling, etc in movable arrangement.

The imaging geometry parameters (α,...,δ) map to different imaging geometry settings. The nature and number of imaging geometry parameters (α,...,δ), and related settings are merely exemplary. There may be more or less, or they may be different such parameters/ settings. In general, the parameters for different imaging geometry settings may correspond to different combination of such parameters. Such parameters may include certain values that pertain to rotation about different axes, and/or lengths, such as one or more shift/translation along one or more respective axis of plural axes. The axes may be of a frame of reference coordinate system. Such imaging geometry settings, and hence parameters combinations, etc, can be set either by the user through said imaging control UI (such as a joystick or other user interface of operating console OC), or may be set automatically by a robot, also envisaged herein. On such setting, the parameters may be translated into lower-level hardware commands. Such hardware command may be propagated via communication infrastructure, such as bus system or other, to respective machine control units of one or more actuators AC. The one or more actuators AC effect the respective imaging geometry re-sets which may cause motion, rotation or both along or about various one or more spatial axes, such as roll, pitch, yaw, etc. Spatially encoding sensors in conjunction with such actuator AC may be used to track the change in imaging geometry gt. Thus, there is an inherent functional dependency of the imaging geometry on time as the procedure unfolds, *gt :* =*g(t).* The projection imagery *λt* acquired that the different imaging geometries is then itself a function of both, respective imaging geometry and time *t,* λ=λ *(g,t).* And it such time series *λ =λ (g, t)* which is received as the said live action feed and displayed as video data on the display device DD for user to monitor device E-based procedure. As said before, displaying of one or more frames as still imagery from among frames *λ =λ (g, t)* is not excluded herein, and some user prefers still imagery over video, at least at certain phases of the procedure.

Fig. 2B) shows a schematic representation of imaging geometry phase space ***G̅*** which may be conceptualized as a high dimensional volume in a data space, such as ${ℝ}^{n}$. For simplicity, a 3D phase (*n=3*) space ***G̅*** is shown, but it will be understood that dimension *n* may be much greater in reality. Different imaging geometries g may be identified as points on or in such a volume ***G̅***, such as a double end-capped cylinder as shown in the Figure, as may pertain to most C-arm imagers, in particular to the DoFs of the C-arm gantry GT. Phase space ***G̅*** may have different representations for other imaging apparatus, and the manner in which their imaging geometries can be adjusted, but in general, the changes over time of imaging geometry g(t) may be conceptualized as paths of points in phase space ***G̅***, such as the exemplary 1D paths in or on 3D cylinder phase space ***G̅*** as shown in the Figure. The paths may be thought to be parameterized by imaging geometry parameters (α,...,δ).

Earlier, in existing systems, two paths *p,q* were to be distinguished: a working path *p,* and an acquisition path *q,* and possibly a changeover *T* between the two. In other words, whilst acquiring projection imagery *λt* for image guided support, that is for navigation, deployment etc, the imaging geometry was adjust, its sole purpose being to follow the procedure and observe the region of interest or any other locality R that needs consideration at any given point in time. This was the work path *p* because it was determined by the work that is to be done, such as the procedure. In other cases, if the user in such existing system had wished a reconstructed image *m,* the imaging geometry needed to be changed over, eg during the transition phase *T,* into a different initial geometry setting, and then perform there a dedicated acquisition path *q,* in general an arc around the region of interest or any other in order to acquire tomographic imagery as needed. Such dedicated path *q* and the changeover *T* amounted to a deliberate choice by the user and called for user action, which was potentially a distraction. This is because earlier users may had to specifically operate the control interface or other user interface components to effect the acquisition path *q.* In particular, this had meant that the work path *p* was abandoned, at least for a time, in favor of the dedicated acquisition path *q.* During the acquisition path *q,* the projection imagery collected in scanning along said path *q,* were not displayed as they as did not as such relate to procedure, its current phase, unlike the projection imagery collected along work path*p*. Such changeover *T* from path *p* to path *q,* and the scanning along path *q,* was also cumbersome, as the movement of the C-arm GT meant user and/or equipment had to be moved out of the way to preclude collision or other harm.

The proposed set-up is a radical departure from this in that there is no more such requested dedicated acquisition path *q* for obtaining tomographic imagery *m* . This concept of acquisition path *q* is abandoned herein. Thus, there is also no more transition *T* needed from work path to another path *q* for the purpose of acquiring projection data for reconstruction. There is solely the work path, dictated by the procedural needs to provide 2D projection imagery-based guidance. And if, one may say by random, it happens that there is a spatial region R (which may well be different from ROI), in respect of which sufficient projection imagery happens to have accumulated in memory over the work path *p*, and/or other image quality conditions are met, only then is an image *mt'* reconstructed at that point t', based on the accumulated projection imagery *λ*, and is then displayed. Such image quality condition(s) may be based on whether the accumulated projection imagery *λ* happens to provide sufficient spatial 3D definition (which will be described in more detail below) of any region R in image domain. Thus, the proposed setup allows a fully automated background tomographic reconstruction initiation and displaying, which allows the user to wholly and solely focus on the work path related matters.

Of course, nothing herein excludes the possibility that the user still has the option to issue as request for a dedicated acquisition path *q,* for particular spatially located tomographic imagery, if and when needed. However, it is expected in the proposed set-up that such a request is rarely used, but can still be done, in addition to the quasi-randomized tomographic reconstruction *mt* that may "pop up" on the way. These quasi-randomized tomographic reconstructions *mt* as proposed herein are reconstructed "on-the-go" as it were, from projection imagery which happens to have accumulated, over to course of the work path *p*, in a buffer or other memory space, as will be discussed herein in more detail. Thus, such quasi-randomized tomographic reconstructions *mt* are "injected" at times (when quality check is passed), for additional 3D insight, supplementing the displayed 2D projection imagery collected in the work path. The 3D reconstructions *mt* have been referred to as quasi-random. This is because a good proportion of procedures may go down as expected, so there may be some regularity on which tomographic imagery *mt* tend to come up. Having said that, each patient and procedure is a case on its own and may well differ from others. Certain anatomic variations may be encountered among other irregularities in some procedures, thus leading at times, to unexpected reconstructions being done and displayed, in an, to the user, apparently randomized fashion. Thus, the proposed setup may be understood as work path dependent "rambling mode" of reconstruction.

It will be appreciated that Fig. 2B is highly schematic for the sake of ease of representation. The paths *p,q* are not confined to level surfaces. Paths across level sets are possible. Also, if all spatial aspects are accounted for, imaging geometry phase space ***G̅*** is in general higher than 3D, such as 6-dimenional, or higher still. In addition, the paths are shown as continuous, which may not necessarily always be the case. Thus, even for the work path, it may not be the case that necessarily at each and every point along the path respective projection imagery is actually acquired.

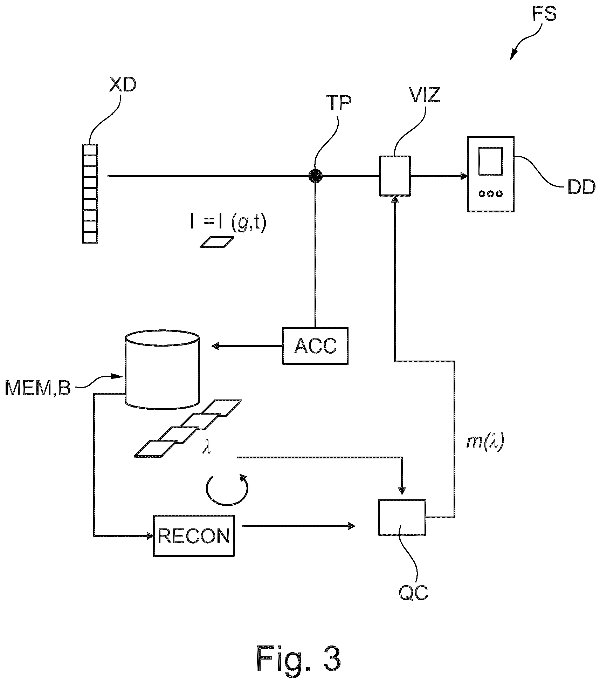

Reference is now made to Fig. 3, which shows a block diagram of components of the facilitator system FS. The facilitator system FS may be a software component such as a process (preferably a background process) that runs on a computing system CS of any type. The computing system CS or the facilitator FS may be a single computational node, or plural computational nodes, such as in a distributed computing setup. The facilitator system FS of the computing system CS or any other on which the facilitator system FS is run as service, process etc, may include processer circuity such as a CPU or a more dedicated chipset such as GPU or any other. The processer circuity has access to memory, volatile and/or non-volatile and a bus system for communication with components such as memory controller, the visualizer (such as graphics card) or components of the facilitator system FS as will be now described below. The bus system may be of parallel or serial architecture, or may be partly both. A PCIexpress architecture may be preferred for responsiveness, but other communication setups are not excluded.

The live action feed projection imagery *λ* =*λ(t,g)* is a function of imaging geometry g used at time *t* at which the respective frame *(t,g)* is acquired. The live action feed projection imagery *λ =λ(t,g)* is acquired at imager's DAQ system, in particular at its X-ray detector XD. It is digitized and optionally pre-pre-processed. The so digitized and optionally pre-processed live action feed projection imagery *λ =λ(t,g)* (referred herein simply as *"projection imagery*/*images*/*image"* or *"frame(s)" λ* or *λ(t,g)* ) is streamed, via suitable communication infrastructure COM and interfacing, to display device DD as facilitated by visualizer VIZ. The projection imagery *λ* is detected at detector XD and visualized over the course of work path *p* as explained above. During streaming, the projection imagery *λ* is tapped off by an accumulator functionality ACC at that tap point TP in the communication infrastructure COM or within the DAQ or elsewhere. The accumulator functionality ACC causes the so detected projection imagery, as such intended for visualization for image guidance over the course of the on-going work path, to be intercepted and stored in a computing memory MEM such as a buffer B. The accumulator functionality ACC may be implemented as an event-driven memory controller, or other. An event handler (not shown) is listening over the communication infrastructure COM and/or in DAQ circuitry for detection of projection frame *λ*, and once detected it not only streamed, but a copy of the projection imagery *λ* is diverted for storage. Such data storing request is repeated during on-going streaming, thereby accumulating the series for projection imagery *λ*. Preferably, high speed memory components may be used for storage, such as volatile memory, such SDRAM, Caches, or other. HBM interfacing may be used, or any other may be used. In particular, volatile memory is used to store the 2D frames λ as they come in, and whilst they are being displayed one after the other in the video feed of screen of display device. Alternatively, and or in addition, non-volatile memory may be used for storage, although this may cause some latency, but comes at the benefit of permanent storage, which may allow some off-line analysis or processing, as required.

Preferably, the projection imagery *λ* is stored with associated metadata such as its respective per frame imaging geometry information *g.* Such imaging geometry information *gt* or specification relates to the imaging geometry used for acquisition of the respective frame *λt*, such as angular, rotation etc, such as some or all of the above mentioned imaging geometry parameters (α,...δ). In addition, a time stamp *t* (which is the acquisition time) is also stored.

The so stored metadata, the projection imagery *λ* and their metadata, may then may be used for evaluation in order to assess whether or not to cause a reconstruction via reconstructor RECON of tomographic imagery *m*, and hence its displaying on display device, or on other display device. This assessment/quality check may be done by an IQ component QC, referred to herein simply as the "quality checker QC". Such image quality check may be done based on one or more of the above mentioned IQ conditions. Its operation may be 1-stage or 2-stage, as mentioned above. Operation may be in image domain or projection domain, or may be in both, or may dynamically change between image domain and projection domain. Operation solely or largely in projection domain may be preferred as this allows avoiding expending compute resources on as may turn out post check, subpar tomographic imagery, ultimately not worthy of displaying. Having said that, a half-way compromise is contemplated herein in some embodiments. In such embodiments, back-projection operation(s) may be sued for some quality checks in imaging domain, without committing compute resources for a full-scale reconstruction, if IQ check fails for a particular set of currently stored projection imagery *λ*.

If IQ check does fail in whichever embodiments, different retention policies may be used. For example, the currently accumulated set may be erased from memory, and new projection imagery *λ* is accumulated, and so forth. A new set *of* projection imagery λ is then re-accumulated and the IQ check is then done on the new set, and so forth. Alternatively, even when IQ checks fails, at least some of the subpar projection imagery *λ* is still retained and used together with newly acquired projection imagery *λ*, until IQ check is passed. This may be of benefit when iterative reconstruction is used. Earlier reconstructed imagery, even when, as yet subpar and not displayed, may be still re-used as new initial data for a new reconstruction, now also including some newly accumulated projection imagery *λ*. Thus, the earlier, possibly subpar image *m,* may be refined eventually into a possibly IQ pass image *m',* which is then displayed. Such reuse of prior data, in particular with iterative reconstruction, may also be done with earlier reconstructed imagery that did pass the IQ check.

Reconstructor RECON may run a suitable tomographic reconstruction algorithm, such as filtered back-projection (FBP), algebraic, iterative, statistical or machine learning based (such as neural network based, in particular CNN based). Reconstructions as such is a computationally demanding process. And it is mainly envisaged herein not to run such reconstruction, unless the quality check by a quality check component QC affirms that a reconstructed volume *m* of a minimum image quality threshold can be achieved. If it can, reconstruction is initiated based on select, not necessarily all, projection imagery accumulated thus far. Thus, the reconstructor imitates reconstruction by reconstructor RECON, once instructed by quality check component QC on a check pass event.

In embodiments, automated selection may be made from the accumulating projection imagery by quality checker QC. The selection is done so that the selected projection imagery *λ* pass the *IQ* check, or that imagery reconstructable from the selection has, or is likely to have, a required IQ level. In preferred embodiments, only when IQ check is passed, is the reconstruction actually fully done until convergence is achieved. Thus, only then does reconstructor RECON access the (selected) projection data as held in memory and starts reconstructing by applying the applicable tomographic reconstruction algorithm to the selected or held data *λ*. Once reconstructed, the so reconstructed image *m* may be stored, processed, but more importantly, may also be visualized on the same display device as the projection imagery or on a different display device, as needed. Reconstructor RECON may proceed iteratively, such as when iterative tomographic reconstruction algorithm is used. In addition, the above-described interaction between reconstructor RECON and quality checker QC may proceed iteratively, as indicated by the curved arrow. The IQ check as administered by quality checker QC may be based on data derivable from the stored projection imagery *λ*, and/or on the projection imagery *λ* as such, and/or on the metadata *g,t* stored in association with the stored projection imagery *λ*.

The reconstructor functionality RECON may use any one of otherwise known tomographic reconstruction algorithms, in particular, but not necessarily those tuned to cope with sparse data, as there may not be enough projection images acquired over a complete 180° arc as may be ideally need for full 3D insight. For better responsive, the reconstructor RECON can be run on processor circuitry capable of parallel processing, such as on those of a multi-core design. GPUs or any other may be used. Computational operations, such as forward -projections and/or backward-projection operations (on which more below) and data updating, may be formulated along SIMD principles. For example, dot-product or matrix multiplications may be used in order to facilitate any one or more of the said computational operations involved in the reconstruction algorithm. Reconstruction algorithms of the iterative type may be preferred herein.

The reconstructed imagery *m=mt* may be a function of time as in different periods during the procedure, a different set of projection data λ may accumulate, that may relate to different regions R, ROI in image domain. On occasion, it may happen that the image quality condition(s) as applied by quality checker QC is met, and a reconstruction is then automatically initiated, and its result *mt* is displayed. Over the course of the work path *p*, other projection data is acquired and accumulated at time t'>t. This newly accumulated projection data *λt*' may happen to pertain to the same region of interest as reconstructed in earlier image *mt.* In such case, the earlier reconstructed image *m,* that was or is still being displayed, may now be used as a new starting point, or new initial data, in the next reconstruction from newly accumulated projection data *λt*', provided, that, again, the IQ quality condition/IQ policy as administered by quality checker QC is met. Thus, the current image *mt* is refined by inclusion of new spatial information from further in-memory accumulated projection images *λt*. Thus, as a result of the new reconstruction, the current image *m* is refined. If such refinement is done whilst the current tomographic image *m* is being displayed, the user may actually "see" on screen DD how the currently displayed volume *mt* is dynamically adapted, that is, morphed into, the refined version *mt'.* The refined image *mt'* may have improved resolution or other gains in image quality.

At other times *t*", over the course of the work path, the newly accumulated projection imagery *λ* may relate to a different region than does the current image *mt.* At that point then, the current image *m*' may be abandoned. If it is still displayed, it may be removed from display, such as at once or it may be phased out gradually. Alternatively, user can request removal of displayed visualization. User can "click it away" for example. The outdated image *mt* may then be replaced by a new image *mt",* reconstructed on IQ check pass from newly accumulated projection imagery *λ*". Thus, the new image *mt"* may now pertain to a different part/phase of the procedure for example. Thus, the facilitator FS may facilitate occasional quality driven 3D imaging in an automated manner, where reconstructed results *mt,m,tmt"* are "injected" when the IQ is right. Thus, facilitator FS may be operable repeatedly at different periods of time from the beginning of the procedure to the end, if needed. Facilitator FS may be operable as background process to quasi-continuously monitor IQ quality in respect of imagery reconstructable from the set or a subset of the currently in-memory accumulated projection imagery *λ*, and may cause their reconstruction, if IQ standard is met, and then effect displaying of such reconstructed imagery. Once reconstructed, and even if no longer displayed, tomographic imagery *m* may be committed for permanent storage in non-volatile memory. Thus, at the conclusion of the procedure, there may possibly be plural such tomographic images stored in non-volatile memory. Such permanently stored imagery *m* may be used for later analytics, training or review/audit, etc. On the other hand, projection imagery *λ* is generally not stored permanently, but this can still be done, if needed. The projection imagery *λ*, although accumulated in memory, is in general not retained and may be expunged, either earlier in the procedure, or once reconstruction happened, or when IQ is not met, or at conclusion of the procedure, etc.

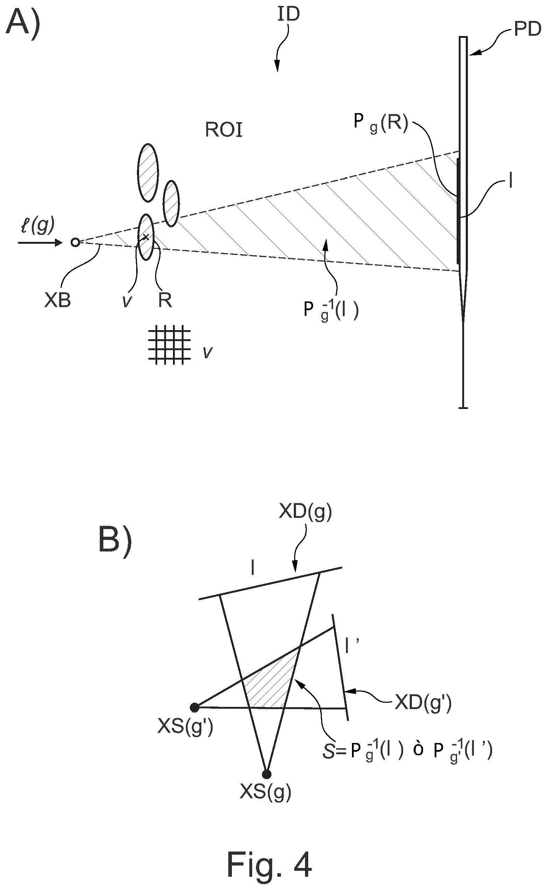

Reference is now made to the schematic drawings in Fig. 4A), B) which show forward- and backward projection operations, as may be used herein in particular, but not only, in iterative reconstruction algorithms by reconstructor RECON, and/or in some quality checks by quality checker QC.

Fig. 4A) illustrates forward- and backward projection along a given projection direction *ℓ* as per a current image geometry setting g. The dark dot on the left in the Figure represents the position of focal spot of the X-ray source XS, and opposite thereto across examination region/image domain ID, the vertical line schematically represents in side-elevation view the imaging surface of the detector XD. This surface, such as a plane, may be made up of X-ray sensitive pixels arranged in a 2D layout. The detector surface situatable at different positions in space (only once such position is shown in the snapshot that is Fig. 4A)) represents the projection domain PD. In distinction, the portion of 3D space in between source XS and detector XD, within which at least part of the imaged subject resides during imaging, is the image domain ID. As said earlier, the image domain ID is conceptually made of a grid of voxel positions v. Broadly, reconstruction, in particular of the tomographic type, is the computational task of determining slice imagery through the examination regions, and hence through subject. This is implemented by reconstructor RECON. A tomographic volume can be thought of as being made up of plural such slices. In the following, "image *m*" may relate to such a given slice, or the volume. In reconstruction, the voxel positions v are populated with image values, based on the projection data, using back projection and, in iterative reconstruction algorithms, forward projection operations.

In the acquisition of projection imagery, intervening materials that make up a region R in the voxelized image domain ID occupy certain of the voxel positions v, and the projected beam projects this spatial information onto the imaging surface (the projection data) as 2D spatially distributed projection data *Πg(R)* ⊂ *λ,* thereby losing some depth information in how the material is distributed along *ℓ.* The forward projected *Πg(R)* pertains to projection information receivable in the field of view, and forms part of the projection data Thus, forward projection is a mapping operation *Π* going from image domain ID *M* to protection domain PD *Λ, Π : M -> Λ* ⊂ *Πg(R).* The reverse of *Π* is back projection operation *B: Λ* ->M. Unlike the forward projection *Π,* which is a many-to-one mapping, the back projection B is a one-to-many mapping. This operation results in a subset in image domain, Π_{g}⁻¹ (λ) ⊂ *M.* Fig. 4A) illustrates such a back projection "cone" (in the general sense) in respect of projection data *λ* on the detector XD's surface. This cone includes all voxels in *M* that are projectable into given projection frame *λ*, given the imaging geometry *g,* and hence in particular projection direction *ℓ* and width of beam. The back-projected cone Πg⁻¹ (λ) may be understood as the pre-image (in the algebraic or set theoretical sense) of the forward projection operation *Π .*

Fig. 4B) illustrates the operation IQ checker QC in embodiments where such back projection operation may be used in order to assess IQ/viability of a reconstruction, given a sets *λ,λ'* of frames of projection data acquired at different geometries *g,g*' as has accumulated in memory over the work path.

The back projections, only two *Π_{g}⁻¹ (λ), Π_{g'}⁻¹ (λ'*) are shown in Fig. 4B), but there may be more, form an intersection region *S*, which could be reconstructed using merely two projections in a sparse reconstruction algorithm. The more projections are acquired, the more back projection cones there are and, if a certain number, say 3 or more are intersecting forming a region R in image domain, this could then be then reconstructed at a reasonable image quality. Other quality indicators based on the time stamp *t* and/or the image geometry meta data g may also be considered herein, and also internal IQ of the projection frames themselves, such motion artifacts, contrast, etc. It will be understood that Fig. 4B) is merely for illustration as merely two cones at different imaging geometries that give rise to different projection directions, are always intersecting by definition. However, for three or more imaging geometries such common intersection for three or more cones is not a necessity. Thus, one IQ condition that may be enforced by quality checker is to check whether there are three of more frames at different imaging geometries *g,g',g''* say, that do form a common intersection *S* in image domain *M*. If yes, a reconstruction *m* may be initiated based on the checked three or more projection frames *λ(g)*, *λ'(g'),λ"(g"*) in the currently accumulated set of projection data. Thus, the earlier mentioned selection from of frames from *λ* may be based on considering their back projections intersecting. As said, further IQ quality checks may be run in addition on the frames in 2D, such for motion artifacts, sufficient contrast, etc. Only when all IQ checks are passed is reconstruction sanctioned by IQ checker QC, and image *m* is releasable for displaying. However, in some simpler embodiments, IQ checker QC may be envisaged that merely does such back-projection based checks for a selection of at least 3 frames at different g's. Such check may be sufficient for some cases. However, back projection check in conjunction with in-projection-frame-contrast check may be called for, all the more so because of the proposed randomized reconstruction. In the proposed randomized reconstructions, it may be likely that, at times, the radiation energy used for irradiation may not be ideal for a given region R, especially if this region is not as such of interest, despite it being seen, by random, in a sufficiently defines "3+" intersection in image domain ID. Whether or not there are geometric loci that form such 3+ intersection S can be established from the imaging geometries, such as projection direction and collimation, etc. CAD or analytic geometry software packages can be used for this.

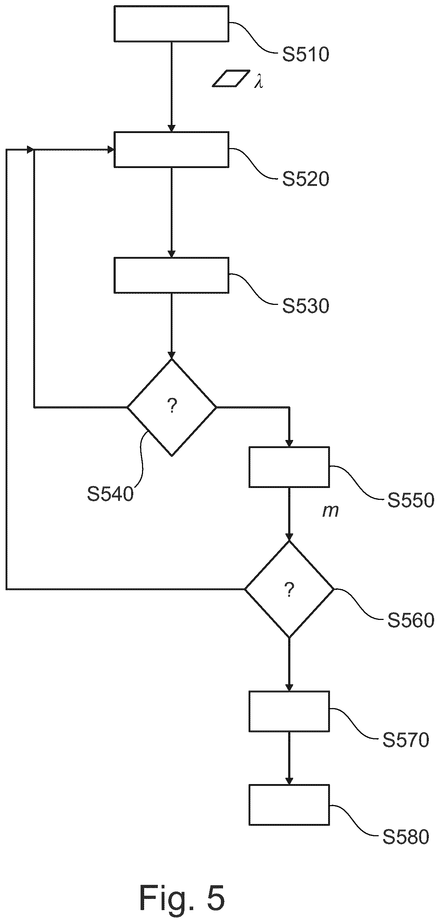

Reference is now made to Fig. 5 which shows a flow chart of a method of facilitating a projection versus 3D reconstruction imaging as may be used in the context of an image value procedure based on projection imagery acquirable by interventional X-ray imager apparatus such as C or U-arm type as discussed above. It will be understood however that whilst the below described steps may be used to implement a system as described above it is not necessarily confined to the architectures discussed above but may be understood instead as a teaching in its own right.

At step S510 a projection data live action feed at different imaging geometries, in particular from different projection directions, is acquired by X-ray imaging apparatus, such as C-arm or other. The imagery may be fluoroscopic frames or angiograms. The imaging geometry settings and the timing of acquisition may depend on procedure which is image-guided by the projection data. The imaging geometries may amount to rotations about one or in general more than one (different) axes in image domain, The rotations may not necessarily be confined to a plane in 3D or be helical. The imaging geometries may be adjusted in an irregular pattern, the user's prime concern being to have the acquired stream of 2D projection imagery *λ* provide image-based guidance for any current local requirement of the procedure, and such locales/regions may change over the work path/procedure as it proceeds of plural phases (navigation, and then deployment of a tool/device, for example).

Specifically, the purpose herein for the acquiring S510 of the projection data/imagery *λ* this is to provide image-guided support for the procedure, such as intervention, eg PCI or other as mentioned above. One such purpose in this connection may be is to follow (panning) in particular one or more tools E that are introduced into subject PAT and threaded or otherwise advanced internally towards ROI, or to follow state changes of tool E, eg, once at ROI, such as repositioning, expansion, etc. As said, the projection imagery may be acquired at different imaging geometries that is, with different FOVs, in particular along different projection directions. This may involve rotation of imager's gantry and hence of source and detector about different portions of image domain. Multiple rotations around possibly different axes may be done. Such rotation(s) may not be confined to a plane and may neither be helical. in general, the imaging geometry changes may be irregular. They can be represented as points in phase space forming work path as per Fig. 2b) above.

The imagery so acquired is streamed, preferably in real-time, at step S520 to a display device for display/visualization there. Thus, the acquired data *λ* is displayed there as video (or still imagery), thereby providing the said-image guided support.

Whilst so the so acquired projection data is acquired and displayed, such projection data is accumulated S530 in a memory by storing the projection data frame as a series of projection frames in said memory. Volatile memory may be used for better performance, but swap outs into non-volatile memory is not excluded, and neither is upfront storing in such non-volatile memory. Preferably, the projection data *λ* is so stored in memory alongside meta data, such as time stamp (time of acquisition) and/or applicable imaging geometry used for the respective frame. The storing may happen during displaying, and this is preferred, but may be done shortly before or after such displaying.

At step S540 first IQ quality check is done on the accumulated projection frames *λ*. This check may be done whilst streaming is ongoing. A selection from the accumulated projection frames *λ* may be made, based on such first quality check.

The selection may be based on proximity of acquisition time and/or backprojections of the frames. Temporally proximal frames may be preferred. Alternatively, or in addition to such time stamp driven check, it may be checked whether three or more backprojections from respective three or more frames at respective three or more different imaging geometries (three or more different projection direction) intersect in 3D. See Fig. 4 above. Other IQ conditions may be checked in addition or instead, such as low motion artifacts and or high contrast. It may be checked whether the stored projection imagery is spatially sufficiently spread, eg, are angularly spaced apparat. Angular or other thresholding may be used. If more conditions are checked for, the outcomes may be considered in combination such as thresholding a common score or other metric. A logic may be uses to process multiple IQ conditions. A conditional logic check may be done processing from one level to the next, only when there is pass in the previous level. This allows saving CPU time. The quality check produces an overall result which is either IQ check passed or not. If no pass, the next selection is considered and/or newly accumulated data is checked. If there is pass, the IQ pass selection (or all currently accumulated frames are passed on to the next step. The quality check preferably results in there of more frames that are deemed singly or in combination as of sufficient IQ (deemed "IQ-passed" for short).

Plural IQ measures/score/metrics as may be used herein. They may be configured to measure any one of more of: i) number of projection mages that can be used, their spatial spread (depending on the angular variance in which the projection images are taken), ii) the quality of the images itself (contrast, etc), iii) possible movements of the patient.

Whilst some of the quality measurements may be done in projection domain, some may be done in image domain. In some embodiments, the image domain quality check may be done during the reconstruction, in particular when using iterative reconstruction algorithm that may involve repeated application of forward and backward projections (Fig. 4B). Thus, image domain quality check S560 may be part of the reconstruction step S550. For example, in the course of the reconstruction, forward projections of the intermediate reconstructions are compared to the underlying projection data and similarity measures such as normalized gradient fields, SSIM (structural similarity index measure), Dice coefficients of overlaps are used. If there is no good correspondence as may be established by thresholding, the reconstruction is aborted but may possibly resume latter once better quality happens to accumulate.

Presence of artifacts may be determining in the image *m* or in the projection data. This could be done by using ML models, such as neural network based model trained for artifacts or by conventional image processing.

Another QM may measure the spatial spread of the projection frames. The better the spread, the better the quality. Thus, the selection of frames may be based on such spread. The imaging geometry metadata may be used to establish such spread. This measure may be used in conjunction with the number of different projection images available. The spread may be established by computing for example the average of and the corresponding difference between the angulation of the C-arm for these images.

At such next step S550 a reconstruction is done, in particular tomographic reconstruction in some embodiments, based on the selection of such projection imagery as currently stored and as IQ passed at step S540. Thus, such reconstruction is done only for IQ-passed frames as per previous step S540. Again, such reconstruction operation may be done whilst streaming is ongoing.

At step S560 an optional second quality check is done based on the final reconstructed volume *m.* In image-domain IQ may be checked such as for motion artifacts, sufficient contrast, etc.

If this optional check is a pass, or simply after reconstruction S550, the tomographic volume *m* is released at step S560 for further processing. Specifically, the tomographic image *m* (volume or slice) *m*ay be displayed at step S570, either on the said display device as the projection imagery is displayed, or on a different display device. For example, the reconstruction *m* may be presented as transparent, colored overlay onto the acquired projections used for reconstruction as well as onto the live fluoroscopy stream. Other models of visualization of the 3D imagery *m* is also envisaged, such as any one or more of:-
- displaying the 3D image m colored, where the color indicates accuracy as per the quality check;
- displaying a partial transparent version of the 3D image *m,* where the transparency is linked to the accuracy of the respective part of the image *m.*

Preferably, the displaying S570 of reconstruction may be done automatically, without any user' input or user provides such input to approve such displaying.

Indeed, the above mentioned operations, in particular the reconstruction quality check, may be done as a background process, without disclosing any information at this point to the user. The display is then automatically displayed which appears for the user to come out of the blue once the quality checks so allow.

At step S580 the accumulated projection data in which the displayed image *m* was reconstructed from is expunged from memory, thus freeing up space for newly accumulated projection data. The process flow may then return to step S530 where newly accumulated data is checked whether reconstruction is worthwhile, and so forth.

In the following, further details may be provided in connection with the above steps. For example, if the second stage quality check at step S560 reveals that the reconstructed volume is not pass, then this is then abandoned, and the processes refers back to step S520 to receive more projection data and the above process repeats. However, the low IQ reconstructed volume may still be used as initial data for follow up reconstruction, thus refining the earlier no-pass image *m.*

The projection data acquired is not as such primarily for reconstruction purposes, but for live image guided support to user of the on-going procedure. The reconstructed imagery, if of sufficient quality, is merely produced and displayed herein as "side image product" that may nevertheless provide useful 3D insight, thus complementing the 2D information in the stream *λ*t. Thus, it will be appreciated based on the foregoing, that the reconstructed volumes produced herein may appear to the user as random, as such imagery *m* may focus on any portion of space that happened to have been in the intersection of a sufficient number of back projections based of the projection data acquired for the purposes of displaying information on the progress of the procedure. Thus, in other words, during operation the checking and reconstruction is done whilst the projection imagery stream is receivable and is being displayed to the user. In some such embodiments, the IQ condition may specify a number of frames at different projection directions. Such number may be set to at least three.

The below describes yet more details on the above, in particular on the above reconstruction using sparse reconstruction algorithm S550, and the quality check operations S540, S560.

Quality check at steps S540 or S560 may relate to motion artifacts. Patient movements and alterations of the patient's anatomy due to the procedure may deteriorate the quality of the 3D reconstruction. On way to address this may be as follow: once at least *N* (*N*>=2) different X-ray images *λ,λ*' at different imaging geometries, such as C-arm angles are accumulated, a selection is made from these *N* images is made. Thus, a subset of *N'* images (*N'<N*) is formed, and reconstructed S550 into the 3D image *m.* This selection is done using multiple metrics or selection criteria. In a preferred embodiment the timestamp of the recording, the location, X-ray beam settings (eg, kV, current, filters, etc) are taken into account in the fitness scoring, determining the image selection. Bony landmarks may be used to determine whether there are movements. Such selection based processing may also be done in respect of IQ other than motion artifacts.

In a preferred embodiment, in-image image quality metrics are used on the 2D projection. IQ are inspected on image artifacts for instance caused by local movements or local anatomy alterations due to the intervention. This artifact score may part of a fitness score that is geared to allow predicting IQ in the reconstruction. For example, based on back-projection of the 2D images, the quality of the reconstruction may be predicted. The view with the best quality is displayed, if above a certain image quality (IQ) threshold. For instance, bony landmarks which are well visible in fluoroscopy can be used for this assessment.

In order to address alterations in the patient's anatomy that may have occurred in between acquisitions, one or multiple aspects as per image information in the 2D images may be taken into account, the most compatible images are then selected and passed on for reconstruction.

As to the reconstruction step S550, using the selected projection views *λ* as per S540, a sparse reconstruction can be undertaken. This may be updated once new relevant views accumulate. In more detail, and referring to step S550, a sparse 3D reconstruction algorithm may be used. Prior to applying such algorithm, a pre-processing may be done on the selected projection images. For example, the selected frame of the accumulated data *λ* may be transformed into line integrals (log-domain). Estimates (calibration data) of the current X-ray flux without object (I-zero) may be used for this. Further pre-processing may include filtering for high contrast in-image objects above a certain threshold in the projection imagery. For example, in image contributions of bone or contrast filled vessels in angiographic protocols may be separated from image background e.g. using morphological filter such as top-hat filter, low-pass, or other filtering. Imaging geometry information, such as patient table position, focus/collimation, detector position and/or SID (detector zoom) may be taken into account and only projections with a common field-of-view (FOV) are processed as previously established by quality check step S540. Optionally, different contrast agent filling states may be analyzed, e.g. using machine learning methods. Such in-image contributions and are excluded or are modified to be consistent for further processing.

Thus, the above preprocessing may be understood as an "image equalizer" step, to ensure consistency of the projection data to be reconstructed from. For example, some projections may have been measured whilst contrast agent was present, while others may have been measured whilst no, or a different amount of contrast agent was present. In addition, motion compensation algorithms may be applied to the projection imagery to reduce morion artifacts. A regularized iterative tomographic reconstruction may be performed that favors reconstruction of sparse objects, e.g. simultaneous, thresholded ART ("Algebraic reconstruction technique") eg ("START"), with *L1*-norm regularization, or others. In another embodiment knowledge of the anatomy is taken into account into the reconstruction. For instance, a model of the spine or other part of the skeletal of the human body is used.

As a refinement, the tomographic reconstruction algorithm may include 3D object modelling. Thus, the tomographic reconstruction algorithm may use, as additional input, in addition to the selected, IQ check pass projection data, information based on anatomical model. Using such model as additional input may allow regularizing in particular iterative type tomographic reconstruction algorithms. Such modelling may allow sizing of image structures, such that intermediate results are made to fit the model, using for example forward-projection on intermediate results. Such structures may include known shapes, such as of bone or vessel geometry. This simplifies and speeds up the reconstruction since the image does not need to be built up from "scratch". The modelling may be done based on segmentation for image structure as per the 2D projection images. Eg, a vessel modelling may be done such as for PCI or other. In such 3D modelling, the image-wise pre-processing required by 3D modelling (vessel profile segmentation, bifurcation localization, vessel identification) is performed on the fly and stored together with the selected projection imagery ("views"), readily available whenever 3D modeling is performed.

The following steps may be done for vessel modelling as an example. For some or each 2D projection image selected, any one of more is done using known image processing techniques: -
- segmenting for main vessels;
- extracting by in-image measurements their diameter profiles;
- anatomically identifying each main segment, eg using an atlas
- identifying relevant one or more landmarks such as vessel bifurcations

Thus, for vessel modeling, one may model the vessels as tubes with bifurcations ,etc. and use this as prior knowledge in the reconstruction.

For some or each newly selected view from newly accumulated data *λ*, a criterium is computed to determine if 3D modelling is possible, that is, if sufficient spatial data has accrued as per the accumulated projection data. This criterium includes measurements such as any one or more of:
- contrast of the observed vessel;
- quality of the vessel segmentation (continuity, regularity);
- number of matching views with matching cardiac phases - ECG readings may be used as measured during imaging with suitable gating; and
- sufficiency of angulation coverage (at least two views, more than 30 degrees apart), or others.

Once the sufficient views and material are gathered as per the above pre-processing, 3D modelling can be achieved. 3D modelling may be based on pairing of the different segmented vascular trees. For this paring, one or more of the following correspondence may be exploited: -
- identity of the vessel segments;
- bifurcation correspondence;
- epipolar constraints;
- diameter correspondence; or other.

Once paired, a 3D model is built, based on the minimization of elementary profiles such as an elliptical profile for at least two views. 3D modeling, in particular for vessels, is described for example in S Çimen, et al. in "Reconstruction of coronary arteries from X-ray angiography: A review", published in "Medical Image Analysis", vol 32. pp. 46-68 (2016).

The components of the facilitator system FS may be implemented as one or more software modules, run on one or more general-purpose processing units PU such as a workstation associated with the imager IA, or on a server computer associated with a group of imagers.

Alternatively, some or all components of the facilitator system FS may be arranged in hardware such as a suitably programmed microcontroller or microprocessor, such an FPGA (field-programmable-gate-array) or as a hardwired IC chip, an application specific integrated circuitry (ASIC), integrated into the imaging system IA. In a further embodiment still, the facilitator system FS may be implemented in both, partly in software and partly in hardware.

The different components of the facilitator system FS may be implemented on a single data processing system. Alternatively, some or more components are implemented on different processing system, possibly remotely arranged in a distributed architecture and connectable in a suitable communication network such as in a cloud setting or client-server setup, etc.

One or more features described herein can be configured or implemented as or with circuitry encoded within a computer-readable medium, and/or combinations thereof. Circuitry may include discrete and/or integrated circuitry, a system-on-a-chip (SOC), and combinations thereof, a machine, a computer system, a processor and memory, a computer program.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above-described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, is presented wherein the computer readable medium has a computer program element stored on it ,which computer program element is described herein.

A computer program may be stored and/or distributed on a suitable medium (in particular, but not necessarily, a non-transitory medium), such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs (set in brackets "()" in the claims) should not be construed as limiting the scope.

## Claims

1. System (SYS) for imaging, comprising:
a projection image accumulator (ACC) causing projection imagery (λ) acquired by an interventional X-ray based imaging apparatus (IA) to be accumulated in a memory (MEM) whilst at least some such projection imagery (λ) is being displayed on a display device (DD) for image-based guidance of a user in performing a procedure in respect of a region of interest; and
a reconstructor (RECON) capable of reconstructing at least one tomographic image *(m),* based at least on some of the accumulated projection imagery.

2. System of claim 1, wherein the system operates in the background during an imaging session with image-based guidance.

3. System of claim 1 or 2, wherein the said reconstructing is ongoing whilst some of the projection imagery is so being displayed.

4. System of any one of claims 1 to 3, wherein the reconstructed imagery *(m)* is displayed once an image quality, IQ, condition is met as establishable by an IQ checker (QC).

5. System of claim 4, where the said IQ condition is based on back projections of the stored projection imagery and/or is based on segmentation of the projection imagery.

6. System of any one of the previous claims, wherein the reconstructed image is of a region (R) other than the ROI.

7. System of any one of the previous claims, wherein any one of initiation of i) reconstruction and ii) the displaying of the reconstructed image is done automatically.

8. System of any one of the previous claims, wherein different plural such images are reconstructed at different field of views, FOVs based on different sets of such stored projection imagery.

9. System of any one of the previous claims, wherein imaging geometry is adjusted for acquisition of the projection imagery.

10. System of any one of the previous claims, wherein further projection imagery is accumulated, and the reconstructed tomographic image is refined by accounting for said further projection imagery in a follow up reconstruction operation by reconstructor (RECON).

11. System of any one of the previous claims, wherein the imaging apparatus (IA) is a C-arm imager and/or is configured for fluoroscopy or angiography.

12. Medical imaging arrangement (IAR) including system and at least one of: the display device, the imaging apparatus, the said memory.

13. Method for imaging, comprising:-
storing (S530) projection imagery (λ) acquired by an interventional X-ray based imaging apparatus (IA) in a memory (MEM), whilst at least some such projection imagery (*λ*) is being displayed on a display device (DD) for image-based guidance of a user in performing a procedure in respect of a region of interest; and
reconstructing (S550) at least one tomographic image *(m),* based at least on some of the accumulated projection imagery.

14. A computer program element, which, when being executed by at least one processing unit, is adapted to cause the processing unit to perform the method as per claim 13.

15. At least one computer readable medium having stored thereon the program element of claim 14.
